# EUROPEAN PATENT APPLICATION

(11) **EP 3 178 813 A1**
(43) Date of publication of application: **14.06.2017**
(21) Application number: 15198729.4
(22) Date of filing: 09.12.2015
(51) Int. Cl.: C07D 231/04, C07C 67/343, C07C 69/738

(54) **METHOD FOR PREPARING HALOGENATED 3-OXOCARBOXYLATES CARRYING A 2-ALKOXYMETHYLIDENE OR A 2-DIALKYLAMINOMETHYLIDENE GROUP**

(71) Applicant: BASF SE, 67056 Ludwigshafen am Rhein (DE)
(72) Inventor: KLAUBER, Eric George, 67098 Bad Dürkheim (DE); GOCKEL, Birgit, 67063 Ludwigshafen (DE); MAYER, Horst, 67069 Ludwigshafen (DE); FRASSETTO, Timo, 68163 Mannheim (DE)
(74) Representative: Reitstötter Kinzebach

(57) **Abstract**

The present invention relates to a process for preparing compounds of the formula I, 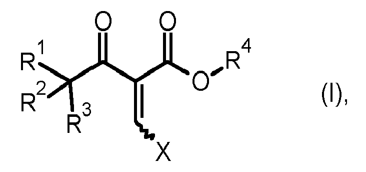 wherein
R¹ and R²
are each independently from one another selected from hydrogen, fluorine, bromine, chlorine and trifluoromethyl,
R³
is selected from fluorine chlorine and trifluoromethyl,
R⁴
is selected from C₁-C₄-alkyl, and
X
is either -OR⁵ or -NR⁶R⁷, wherein R⁵ is selected from C₁-C₄-alkyl, and R⁶ and
R⁷
are each independently from one another selected from C₁-C₄-alkyl,

which comprises the reaction of a compound of the formula II 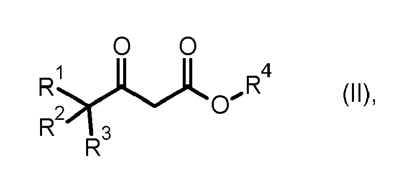 with a reagent of the formula III, 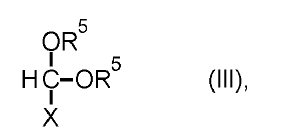 wherein the variables R¹, R², R³, R⁴, R⁵ and X given in formulae II and III have the above defined meanings,
in the presence of a catalyst selected from Brønsted acids, ammonium salts of Brønsted acids and Lewis acids;
wherein the reaction is performed essentially in the absence of a carboxylic acid anhydride.

## Description

The present invention relates to a process for preparing compounds of the formula I, 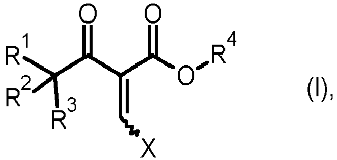 wherein
- R¹ and R²: are each independently from one another selected from hydrogen, fluorine, bromine, chlorine and trifluoromethyl,
- R³: is selected from fluorine, chlorine and trifluoromethyl,
- R⁴: is selected from C₁-C₄-alkyl, and
- X: is either -OR⁵ or -NR⁶R⁷, wherein R⁵ is selected from C₁-C₄-alkyl, and R⁶ and R⁷ are each independently from one another selected from C₁-C₄-alkyl.

Compounds of the general formula I, such as in particular 4,4-difluoro-2-ethoxy-methylidene-3-oxobutanoates, are valuable synthetic intermediates for a number of active pharmaceutical ingredients and fungicidal active ingredients, especially 1,3-substituted pyrazol-4-ylcarboxanilides, as described for instance in US 5,498,624, EP 545099 A1, EP 589301 A1, WO 92/12970, WO 03/066610, WO 2006/024389, WO 2007/003603 and WO 2007/006806. Examples of commercial fungicides for which 4,4-difluoro-2-ethoxymethylidene-3-oxobutanoates serve as key building blocks are sedaxane, fluxapyroxad, bixafen, isopyrazam, benzovondiflupyr and pydiflumetofen.

Halogenated 2-alkoxymethylidene-3-oxobutanoates are typically prepared by reacting the respective halogenated 3-oxobutanoate with triethyl orthoformate in the presence of a molar excess of acetic anhydride which serves as scavenger of the ethanol formed during the reaction. Accordingly, R.G. Jones describes the preparation of ethyl 4,4,4-trifluoro-2-ethoxymethylidene-3-oxobutanoate by condensing ethyl 4,4,4-trifluoro-3-oxobutanoate with 1.5 molar equivalents of triethyl orthoformate and 3 molar equivalents of acetic anhydride over 7 hours at 120 to 140°C to yield 67% of the desired product (R.G. Jones, J. Am. Chem. Soc. 1951, 73, 3684-3686). Very similar processes are still used for producing 4,4-difluoro-2-alkoxymethylene-3-oxobutyrates and other compounds of formula I on a technical scale. However, these processes require the removal of large amounts of acetic anhydride and alkyl acetate after completion of the reaction, rendering them time-consuming and expensive. In addition, the obtained yields and purities are not entirely satisfactory.

WO2013/171102 and J. Jaunzems et al. disclose the preparation of ethyl 4-chloro-4,4-difluoro-2-ethoxymethylidene-3-oxobutanoate by reacting ethyl 4-chloro-4,4-difluoro-3-oxobutanoate with 5 molar equivalents of ethyl orthoformate in the presence of a catalytical amount of the base triethylamine. The ethanol formed in the course of the reaction is distilled off (see J. Jaunzems et al., Organic Process Research & Development 2014, 18(8), 1055-1059). However, the large excess of ethyl orthoformate used in this process has to be tediously removed afterwards during the workup procedure.

M. V. Pyradeina et al. describe reacting ethyl 4,4,4-trifluoro-3-oxobutanoate with an excess of triethyl orthoformate under reflux with simultaneous distillative removal of ethanol. The reaction that was carried out without adding a catalyst resulted in only modest yields of ethyl 4,4,4-trifluoro-2-ethoxymethylidene-3-oxobutanoate (see M. V. Pyradeina et al. Russian Journal of Organic Chemistry 2007, 43(7), 945-955).

It is therefore an object of the invention to provide a process that allows the highyielding and selective preparation of the compounds of formula I cited at the outset, i.e. the halogenated 2-alkoxymethylidene-3-oxocarboxylates of the formula IA and the halogenated 2-dialkylaminomethylidene-3-oxocarboxylates of the formula IB, 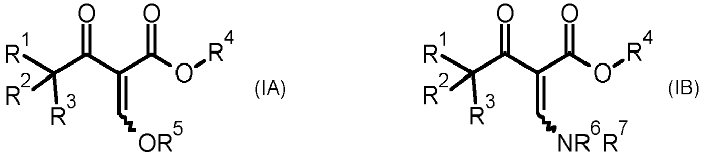 wherein the variables R¹, R², R³, R⁴, R⁵, R⁶ and R⁷ are as defined for formula I, in an economical manner. The process should in particular avert tedious workup measures for removing large amounts of solvents, byproducts and/or unconsumed reactants.

Is has been found that, surprisingly, the compounds of the formula I defined at the outset can be prepared in a simple manner in high yields and good selectivity without requiring costly work-up procedures.

Accordingly, the present invention relates to a process for preparing compounds of the formula I defined at the outset, which comprises the reaction of a compound of the formula II, 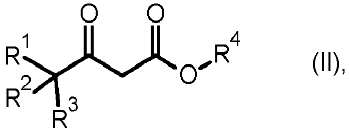 with a reagent of the formula III, 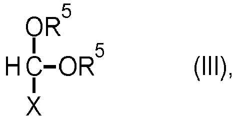 in the presence of a catalyst selected from Brønsted acids, ammonium salts of Brønsted acids and Lewis acids; wherein the variables R¹, R², R³, R⁴, R⁵ and X given in formulae II and III are as defined for formula I, and wherein the reaction is performed essentially in the absence of a carboxylic acid anhydride.

The process according to the invention is associated with a series of advantages. Firstly, it affords the desired compounds of the formula I in high yields and very low levels of byproducts without requiring substantial amounts of a solvent or any supplemental reagents, such as acetic anhydride. In addition, only mild reaction conditions, such as relatively low reaction temperatures, are required. The process of the invention, therefore, allows for the easy, efficient and economical production of the desired compounds of formula I.

By drawing the bond between the moiety X and the methylidene carbon atom in formula I as a wobbly line it is indicated that the compound of the formula I may be present as the Z-isomer I-z, the E-isomer I-e or a mixture of both isomers, 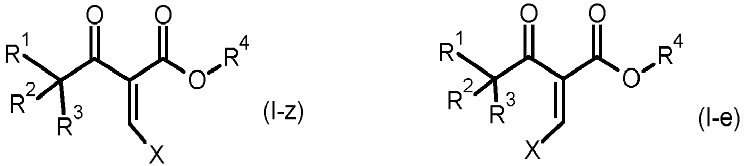 wherein the variables R¹, R², R³, R⁴ and X are as defined for formula I.

It is assumed that in the process of the invention the reaction of the compound of formula II with the reagent of the formula III typically proceeds via the acetal intermediate of the formula IV, 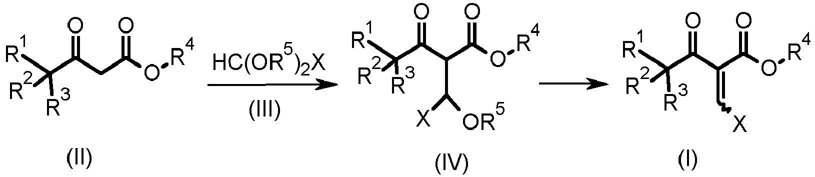 wherein the variables R¹, R², R³, R⁴, R⁵ and X are as defined for formula I.

In the context of the present invention, the terms used generically are defined as follows:
The prefix Cₓ-C_{y} denotes the number of possible carbon atoms in the particular case.

The term "C₁-C₄-alkyl", as used herein, denotes a linear or branched alkyl radical comprising from 1 to 4 carbon atoms, especially from 1 to 2 carbon atoms, such as methyl, ethyl, propyl, 1-methylethyl (isopropyl), butyl, 1-methylpropyl (sec-butyl), 2-methylpropyl (isobutyl) or 1,1-dimethylethyl (tert-butyl).

The expression "essentially in the absence of a carboxylic acid anhydride", as used herein, means that the reaction according to the process of the invention is carried without any carboxylic acid anhydride, such as C₁-C₄-alkanoic acid anhydrides and in particular acetic acid anhydride, being present in substantial amounts; i.e. one or more carboxylic acid anhydrides may only be present in a combined amount of not more than 5% by weight, preferably not more than 2% by weight, in particular not more than 1 % by weight and especially not more than 0.1 % by weight, in relation to the total amount of the reaction mixture.

The compound II used in the process of the invention may be present in the form of the ketone of the formula II, or in the form of its ketal of the formula IIa, its semiketal of the formula IIb or its hydrate of the formula IIc, or in the form of a mixture including all or some of these four derivatives of the compound II in any combination. The ketal, the semiketal and the hydrate of the compound II are of the formulae IIa, IIb and IIc, respectively, 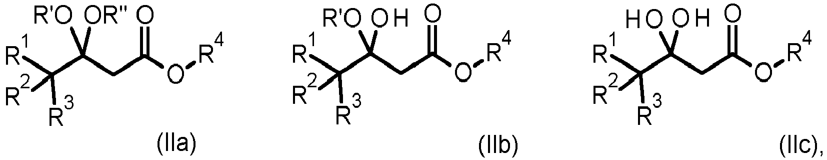 wherein R' and R" are each selected from C₁-C₄-alkyl, an in particular from methyl and ethyl.

According to one embodiment of the present invention the compound of the formula II used as starting material in the process of the invention is essentially pure, i.e. at least 95% by weight, preferably 97% by weight and in particular 99% by weight relative to the total amount of compound II are in the form the ketone of the formula II.

According to another embodiment of the invention the compound of the formula II comprises up to 25% by weight, preferably up to 20% by weight and in particular up to 15% by weight of the ketal IIa, the semiketal IIb or the hydrate IIc, or any mixture of these compounds.

A preferred embodiment of the invention relates to the preparation of compounds of the formula I in which
- R¹: is selected from hydrogen, fluorine and chlorine, in particular from hydrogen and fluorine, and especially is fluorine,
- R²: is selected from hydrogen, chlorine and trifluoromethyl, in particular from hydrogen and trifluoromethyl, and
- R³: is fluorine.

In accordance with this embodiment the variables R¹, R² and R³ in formulae II and IV have the same preferred meanings.

Another preferred embodiment of the invention relates to the preparation of compounds of the formula I in which R¹ is hydrogen or fluorine. In accordance with this embodiment the variable R¹ in formulae II and IV is also preferably hydrogen or fluorine.

A particularly preferred embodiment of the invention relates to the preparation of compounds of the formula I in which R¹ is fluorine and R² is either hydrogen or trifluoromethyl. According to this embodiment compounds of the formulae II and IV are preferred in which R¹ is fluorine and R² is hydrogen, or in which R¹ is fluorine and R² is trifluoromethyl.

Another preferred embodiment of the invention relates to the preparation of compounds of the formula I in which R⁴ is methyl or ethyl. In accordance with this embodiment the variable R⁴ in the formulae II and IV is also preferably methyl or ethyl. According to a particularly preferred embodiment R⁴ is ethyl.

Accordingly, the compound of the formula II used in the process of the invention is preferably selected from methyl 4-fluoro-3-oxobutanoate, ethyl 4-fluoro-3-oxobutanoate, methyl 4,4-difluoro-3-oxobutanoate, ethyl 4,4-difluoro-3-oxobutanoate, methyl 4,4,4-trifluoro-3-oxobutanoate, ethyl 4,4,4-trifluoro-3-oxobutanoate, methyl 4,5,5,5-tetrafluoro-3-oxopentanoate, ethyl 4,5,5,5-tetrafluoro-3-oxopentanoate, methyl 4,4,5,5,5-pentafluoro-3-oxopentanoate and ethyl 4,4,5,5,5-pentafluoro-3-oxopentanoate, in particular selected from methyl 4,4-difluoro-3-oxobutanoate, ethyl 4,4-difluoro-3-oxobutanoate, methyl 4,4,5,5,5-pentafluoro-3-oxopentanoate and ethyl 4,4,5,5,5-pentafluoro-3-oxopentanoate, and specifically selected from ethyl 4,4-difluoro-3-oxobutanoate and ethyl 4,4,5,5,5-pentafluoro-3-oxopentanoate.

Another preferred embodiment of the invention, hereafter called embodiment A, relates to the preparation of compounds of the formula IA by reacting a compound of the formula II with a reagent of the formula III in which the variable X is -OR⁵. Thus, according to this embodiment the reagent III is of the formula HC(OR⁵)₃. Likewise, the variable X in the compound of formula IV is -OR⁵. R⁵ is selected from C₁-C₄-alkyl, in particular from methyl and ethyl, and especially is ethyl.

Accordingly, the reagent of the formula III used in the process of embodiment A is preferably selected from triethyl orthoformate and trimethyl orthoformate, and in particular is triethyl orthoformate.

In the context of embodiment A particular preference is given to compounds IA wherein R⁵ is identical with R⁴, i.e. the variables R⁵ and R⁴ both represent the same C₁-C₄-alkyl group, preferably methyl or ethyl and in particular ethyl. Likewise, according to embodiment A the variables R⁴ and R⁵ in the compounds of formula IV with X being -OR⁵ are preferably identical C₁-C₄-alkyl groups, and in particular are both methyl or ethyl, especially ethyl.

In addition, the halogenated 2-alkoxymethylidene-3-oxocarboxylate of formula IA to be prepared by the process according to the embodiment A is preferably selected from methyl 4-fluoro-2-ethoxymethylidene-3-oxobutanoate, ethyl 4-fluoro-2-ethoxymethylidene-3-oxobutanoate, methyl 4,4-difluoro-2-ethoxymethylidene-3-oxobutanoate, ethyl 4,4-difluoro-2-ethoxymethylidene-3-oxobutanoate, methyl 4,4,4-trifluoro-2-ethoxymethylidene-3-oxobutanoate, ethyl 4,4,4-trifluoro-2-ethoxymethylidene-3-oxobutanoate, methyl 4,5,5,5-tetrafluoro-2-ethoxymethylidene-3-oxopentanoate, ethyl 4,5,5,5-tetrafluoro-2-ethoxymethylidene-3-oxopentanoate, methyl 4,4,5,5,5-pentafluoro-2-ethoxymethylidene-3-oxopentanoate, ethyl 4,4,5,5,5-pentafluoro-2-ethoxymethylidene-3-oxopentanoate, methyl 4-fluoro-2-methoxymethylidene-3-oxobutanoate, ethyl 4-fluoro-2-methoxymethylidene-3-oxobutanoate, methyl 4,4-difluoro-2-methoxymethylidene-3-oxobutanoate, ethyl 4,4-difluoro-2-methoxymethylidene-3-oxobutanoate, methyl 4,4,4-trifluoro-2-methoxymethylidene-3-oxobutanoate, ethyl 4,4,4-trifluoro-2-methoxymethylidene-3-oxobutanoate, methyl 4,5,5,5-tetrafluoro-2-methoxymethylidene-3-oxopentanoate, ethyl 4,5,5,5-tetrafluoro-2-methoxymethylidene-3-oxopentanoate, methyl 4,4,5,5,5-pentafluoro-2-methoxymethylidene-3-oxopentanoate and ethyl 4,4,5,5,5-pentafluoro-2-methoxymethylidene-3-oxopentanoate, in particular selected from methyl 4,4-difluoro-2-ethoxymethylidene-3-oxobutanoate, ethyl 4,4-difluoro-2-ethoxymethylidene-3-oxobutanoate, methyl 4,4,5,5,5-pentafluoro-2-ethoxymethylidene-3-oxopentanoate, ethyl 4,4,5,5,5-pentafluoro-2-ethoxymethylidene-3-oxopentanoate, methyl 4,4-difluoro-2-methoxymethylidene-3-oxobutanoate, ethyl 4,4-difluoro-2-methoxymethylidene-3-oxobutanoate, methyl 4,4,5,5,5-pentafluoro-2-methoxymethylidene-3-oxopentanoate and ethyl 4,4,5,5,5-pentafluoro-2-methoxymethylidene-3-oxopentanoate, and specifically selected from ethyl 4,4-difluoro-2-ethoxymethylidene-3-oxobutanoate and ethyl 4,4,5,5,5-pentafluoro-2-ethoxymethylidene-3-oxopentanoate.

Yet another preferred embodiment of the invention, hereafter called embodiment B, relates to the preparation of compounds of the formula IB by reacting a compound of the formula II with a reagent of the formula III in which the variable X is -NR⁶R⁷. Thus, according to this embodiment the reagent III is of the formula HC(OR⁵)₂(NR⁶R⁷). Likewise, the variable X in the compound of formula IV is also -NR⁶R⁷. Here R⁵ is selected from C₁-C₄-alkyl, in particular from methyl and ethyl, and especially is ethyl, while R⁶ and R⁷ are each independently from one another selected from C₁-C₄-alkyl and in particular from methyl and ethyl. Preferably the variables R⁶ and R⁷ have the same meaning and are both either methyl or ethyl.

Accordingly, the reagent of the formula III used in the process of embodiment B is preferably selected from 1,1-diethoxy-N,N-dimethylmethanamine, 1,1-dimethoxy-N,N-dimethylmethanamine, 1,1-diethoxy-N,N-diethylmethanamine and 1,1-dimethoxy-N,N-diethylmethanamine, and in particular is 1,1-diemthoxy-N,N-dimethylmethanamine.

In the context of embodiment B particular preference is given to compounds IB wherein the variables R⁴, R⁶ and R⁷ are each independently from one another selected from methyl and ethyl. Particular preference is given to compounds IB wherein R⁴ is methyl or ethyl and the variables R⁶ and R⁷ have the same meaning and are both either methyl or ethyl, preferably are both methyl.

In addition, the halogenated 2-dialkylaminomethylidene-3-oxocarboxylate of formula IB to be prepared by the process according to the embodiment B is preferably selected from methyl 4-fluoro-2-dimethylaminomethylidene-3-oxobutanoate, ethyl 4-fluoro-2-dimethylaminomethylidene-3-oxobutanoate, methyl 4,4-difluoro-2-dimethylaminomethylidene-3-oxobutanoate, ethyl 4,4-difluoro-2-dimethylaminomethylidene-3-oxobutanoate, methyl 4,4,4-trifluoro-2-dimethylaminomethylidene-3-oxobutanoate, ethyl 4,4,4-trifluoro-2-dimethylaminomethylidene-3-oxobutanoate, methyl 4,5,5,5-tetrafluoro-2-dimethylaminomethylidene-3-oxopentanoate, ethyl 4,5,5,5-tetrafluoro-2-dimethylaminomethylidene-3-oxopentanoate, methyl 4,4,5,5,5-pentafluoro-2-dimethylaminomethylidene-3-oxopentanoate, ethyl 4,4,5,5,5-pentafluoro-2-dimethylaminomethylidene-3-oxopentanoate, methyl 4-fluoro-2-diethylaminomethylidene-3-oxobutanoate, ethyl 4-fluoro-2-diethylaminomethylidene-3-oxobutanoate, methyl 4,4-difluoro-2-diethylaminomethylidene-3-oxobutanoate, ethyl 4,4-difluoro-2-diethylaminomethylidene-3-oxobutanoate, methyl 4,4,4-trifluoro-2-diethylaminomethylidene-3-oxobutanoate, ethyl 4,4,4-trifluoro-2-diethylaminomethylidene-3-oxobutanoate, methyl 4,5,5,5-tetrafluoro-2-diethylaminomethylidene-3-oxopentanoate, ethyl 4,5,5,5-tetrafluoro-2-diethylaminomethylidene-3-oxopentanoate, methyl 4,4,5,5,5-pentafluoro-2-diethylaminomethylidene-3-oxopentanoate and ethyl 4,4,5,5,5-pentafluoro-2-diethylaminomethylidene-3-oxopentanoate, in particular selected from methyl 4,4-difluoro-2-dimethylaminomethylidene-3-oxobutanoate, ethyl 4,4-difluoro-2-dimethylaminomethylidene-3-oxobutanoate, methyl 4,4,5,5,5-pentafluoro-2-dimethylaminomethylidene-3-oxopentanoate, ethyl 4,4,5,5,5-pentafluoro-2-dimethylaminomethylidene-3-oxopentanoate, methyl 4,4-difluoro-2-diethylaminomethylidene-3-oxobutanoate, ethyl 4,4-difluoro-2-diethylaminomethylidene-3-oxobutanoate, methyl 4,4,5,5,5-pentafluoro-2-diethylaminomethylidene-3-oxopentanoate and ethyl 4,4,5,5,5-pentafluoro-2-diethylaminomethylidene-3-oxopentanoate, and specifically selected from ethyl 4,4-difluoro-2-dimethylaminomethylidene-3-oxobutanoate and ethyl 4,4,5,5,5-pentafluoro-2-dimethylaminomethylidene-3-oxopentanoate.

The reaction of the process according to the invention as described hereinafter is performed in a reaction vessel customary for such reactions. Typically, the reaction is carried out in a semicontinuous or batchwise manner but, alternatively, may possibly also be conducted in a continuous manner. The reaction is preferably carried out under reduced pressure, but may also be carried out under atmospheric pressure or elevated pressure.

Preferably the combined quantities of the compounds of formulae I, II, IIa, IIb, IIc and III amount for at least 75% by weight, preferably at least 80% by weight, in particular at least 90% by weight, and specifically at least 98% by weight of the reaction mixture at the beginning of the reaction, especially if the reaction is conducted in a batchwise or semicontinuous manner.

The reaction of the process according to the invention for preparing a compound of the formula I may be regarded as an aldol-type condensation reaction. The conversion is effected by reacting a halogenated 3-oxocarboxylate of the formula II with a reagent of the formula III in the presence of a catalyst selected from Brønsted acids, ammonium salts of Brønsted acids and Lewis acids.

According to an embodiment of the present invention the catalyst is a Brønsted acid or an ammonium salts of Brønsted acid, in particular one of those Brønsted acids or one of their ammonium salts that are mentioned herein as preferred.

Brønsted acids suitable for use in the process of the invention may be any organic or mineral acid capable of catalyzing the reaction of the process by acting as proton donator.

Preferred Brønsted acids for the process of the invention are selected from C₁-C₄-alkanoic acids, aryl carboxylic acids, oxoacids of phosphor, C₁-C₄-alkyl sulfonic acids, aryl sulfonic acids and cycloaliphatic sulfonic acids. Even more preferred Brønsted acids for the process of the invention are selected from phosphoric acids, C₁-C₄-alkyl sulfonic acids and aryl sulfonic acids.

In this context "C₁-C₄-alkanoic acid" denotes carboxylic acids R-CO₂H wherein the group R is selected from C₁-C₃-alkyl. Examples of C₁-C₄-alkanoic acids are formic acid, acetic acid, propionic acid and butyric acid.

The term "aryl carboxylic acid", as used herein, describes carboxylic acids R^{a}-CO₂H with the variable R^{a} denoting an aryl group selected from phenyl and naphthyl, wherein phenyl and naphthyl may be unsubstituted or substituted with 1, 2, 3, 4 or 5 substituents selected from C₁-C₄-alkyl, chlorine and bromine. Examples of aryl carboxylic acids are benzoic acid, 4-methylbenzoic acid, 2-methylbenzoic acid, 2,4-dimethylbenzoic acid, 4-chlorobenzoic acid, 1-naphthalenecarboxylic acid, 2-naphthalenecarboxylic acid, 2-methyl-1-naphthalenecarboxylic acid, 4-methyl-2-naphthalenecarboxylic acid, 6-methyl-2-naphthalenecarboxylic acid, 1,4-dimethyl-2-naphthalenecarboxylic acid, 1,5-dimethyl-2-naphthalenecarboxylic acid and 5,6-dimethyl-2-naphthalenecarboxylic acid.

The term "oxoacids of phosphor", as used herein, encompasses any acid, which has a OH-group or NH₂ group bound to phosphor, especially an acid having 1, 2 or 3 OH groups or 1 NH₂ group, which are bound to a phosphor atom in the oxidation state III or V. The term "oxoacids of phosphor", as used herein, in particular encompasses the following classes of acids:
- Phosphoric acid, its oligomers and its mono- or di-estersorthophosphoric acid (H₃PO₄), pyrophosphoric acid, polyphosphoric acids, aryl dihydrogen phosphates, such as phenyl dihydrogen phosphate or 1-naphthyl dihydrogen phosphate, alkyl dihydrogen phosphate, such as butyl dihydrogen phosphate or 2-ethylhexyl dihydrogen phosphate, benzyl dihydrogen phosphate and substituted derivatives thereof,
- Phosphonic acid, and semiesters thereof,
- Phosphinic acids, e.g. Aryl phosphinic acids, such as phenyl phosphinic acid,
- Phosphoric amidates, such as diethyl phosphoramidate, dibenzylphosphoramidate or dibenzyl phosphoramidate.

The term "C₁-C₄-alkyl sulfonic acid", as used herein, describes sulfonic acids R^{b}-SO₃H wherein the group R^{b} is selected from C₁-C₄-alkyl. Examples of C₁-C₄-alkyl sulfonic acid are methanesulfonic acid, ethanesulfonic acid, 1-propanesulfonic acid, 2-propanesulfonic acid, 1-butanesulfonic acid, 2-butanesulfonic acid and 2-methyl-2-propanesulfonic acid.

The term "aryl sulfonic acid", as used herein, describes sulfonic acids R^{a}-SO₃H with the variable R^{a} denoting an aryl group selected from phenyl and naphthyl, wherein phenyl and naphthyl may be unsubstituted or be substituted with 1, 2, 3, 4 or 5 substituents selected from C₁-C₄-alkyl. Examples of aryl sulfonic acids are benzenesulfonic acid, 4-toluenesulfonic acid, 2-toluenesulfonic acid, 2,4-xylenesulfonic acid, 1-naphthalenesulfonic acid, 2-naphthalenesulfonic acid, 2-methyl-1-naphthalenesulfonic acid, 4-methyl-2-naphthalenesulfonic acid, 6-methyl-2-naphthalenesulfonic acid, 1,4-dimethyl-2-naphthalenesulfonic acid, 1,5-dimethyl-2-naphthalenesulfonic acid and 5,6-dimethyl-2-naphthalenesulfonic acid.

The term "cycloaliphatic sulfonic acids", as used herein, describes sulfonic acids R_{c}-SO₃H wherein the group R^{c} is selected from C₃-C₁₀-cycloalkyl and C₃-C₁₀-cycloalkyl-C₁-C₄-alkyl, wherein C₃-C₁₀-cycloalkyl in each case is a mono- or bicyclic moiety optionally carrying 1, 2, 3, 4, 5 or 6 substituents that are selected from bromine, chlorine, C₁-C₄-alkyl, or two of said substituents positioned at the same carbon atom represent the oxygen atom of a carbonyl group. Examples of cycloaliphatic sulfonic acids are cyclohexane sulfonic acid and camphorsulfonic acid.

The expression "ammonium salts of Brønsted acids", as used herein, denotes salts obtained by neutralizing Brønsted acids, in particular those mentioned before as preferred, with ammonia or organic amines. In this context organic amines are preferably selected from aromatic amines, such as pyridine or collidine, heterocyclic amines, such as piperidine, 2,2,6,6-tetramethylpiperidine, 2,2,6,6-tetramethylpiperidone or morpholine, aryl amines, such as aniline or 4-methylaniline, secondary and tertiary mixed alkyl-aryl amines, such as N-methyl aniline or N,N-dimethyl aniline, and primary, secondary and tertiary aliphatic amines, such as triethylamine, diethylamine, 1-propylamine or 2-cyclopropyl-2-propylamine, particularly selected from pyridine, collidine, morpholine and trimethylamine, and specifically selected from pyridine and trimethylamine.

Preferred ammonium salts of Brønsted acids for the process of the invention are obtained by neutralizing a Brønsted acid selected from C₁-C₄-alkanoic acids, aryl carboxylic acids, oxoacids of phosphor, C₁-C₄-alkyl sulfonic acids and aryl sulfonic acids, in particular from phosphoric acid (H₃PO₄), C₁-C₄-alkyl sulfonic acids and aryl sulfonic acids and especially from C₁-C₄-alkyl sulfonic acids and aryl sulfonic acids, with ammonia or an organic amine, in particular one of the organic amines mentioned before as preferred.

In a particular embodiment of the present invention the catalyst used in the process of the invention is a either a Brønsted acid which is selected from H₃PO₄, methansulfonic acid, ethansulfonic acid, benzenesulfonic acid, 4-toluenesulfonic acid, 2,4-xylenesulfonic acid and camphorsulfonic acid, in particular from H₃PO₄, methansulfonic acid, benzenesulfonic acid, 4-toluenesulfonic acid and camphorsulfonic acid, especially from H₃PO₄, methansulfonic acid and 4-toluenesulfonic acid, or an ammonium salt of one of these Brønsted acids which is particularly selected from ammonium dihydrogen phosphate, pyridinium dihydrogen phosphate, trimethylammonium dihydrogen phosphate, ammonium methansulfonate, pyridinium methansulfonate, morpholinium methansulfonate, trimethylammonium methansulfonate, ammonium 4-toluenesulfonate, pyridinium 4-toluenesulfonate, morpholinium 4-toluenesulfonate and trimethylammonium 4-toluenesulfonate, and is specifically selected from trimethylammonium methansulfonate, pyridinium 4-toluenesulfonate and trimethylammonium 4-toluenesulfonate.

In a special embodiment of the present invention the catalyst used in the process of the invention is H₃PO₄.

According to an alternative embodiment the Brønsted acid is not added as such to the reaction mixture of the process according to the present invention, but instead is generated in-situ during the reaction by adding water to the reaction mixture. This way the Brønsted acid formic acid is formed via hydrolysis of the reagent III.

Lewis acids suitable for use in the process of the invention may be any compound capable of catalyzing the reaction of the process by acting as an acceptor of a pair of electrons.

Preferred Lewis acids for the process according to the invention are selected from halides of metals and metalloids and derivatives thereof, such as MgF₂, BF₃·OEt₂, BCl₃, AlCl₃, AlF₃, ZnCl₂, FeCl₃, PF₅, SbF₅, TiCl₄, BiCl₃, GaCl₃, SnCl₄ and SiCl₄.

In a particularly preferred embodiment the Lewis acid used in the process of the invention is selected from ZnCl₂, FeCl₃, TiCl₄ and AlCl₃, and in particular is ZnCl₂.

In the reaction of the process according to the invention the molar ratio of the catalyst to the compound of formula II is preferably from 0.00001:1 to 0.2:1, more preferably from 0.0001:1 to 0.1:1, in particular from 0.0005:1 to 0.05:1, and specifically from 0.002:1 to 0.04:1.

In case a Brønsted acid is used as catalyst that is generated in situ by adding water to the reaction mixture, as described above, water is added in an amount of preferably from 0.0001:1 to 0.1:1, in particular from 0.0005:1 to 0.05:1, and specifically from 0.002:1 to 0.04:1.

In a further preferred embodiment of the invention the molar ratio of the reagent of the formula III to the compound of formula II is preferably from 1:1 to 10:1, more preferably from 1:1 to 7:1, in particular from 1:1 to 6:1 and specifically from 1.1:1 to 5.5:1. In a particular preferred embodiment the molar ratio of the reagent III to the compound II is from 1:1 to 3:1, in particular from 1:1 to 2:1 and specifically from 1.1:1 to 1.8:1.

The reaction of the process according to the invention may or may not be carried out in the presence of an additional compound solely serving as a solvent. If such a solvent is used it is typically selected from inert organic solvents, such as aromatic hydrocarbons and halohydrocarbons, for example benzene, toluene, xylenes, cumene, chlorobenzene and tert-butylbenzene, cyclic or acyclic ethers such as diethyl ether, diisopropyl ether, tert-butyl methyl ether (MTBE), tert-butyl ethyl ether, tetrahydrofuran (THF) or dioxane, nitriles such as acetonitrile and propionitrile, aliphatic halohydrocarbons such as dichloromethane, dichloroethane, trichloromethane and mixtures thereof.

In a preferred embodiment the reaction of the process according to the invention is carried out in the absence of a compound that solely serves as a solvent. In fact, the compound of the formula III not only serves as a reagent but in addition as a solvent, in particular if it is used in a 2- to 20-fold molar excess relative to the compound of the formula II in accordance to the previously mentioned preferred embodiment.

In case an additional compound that solely serves as a solvent is used in the reaction of the process according to the invention, it is present in the reaction mixture in an amount of typically not more than 20% by weight, preferably not more than 10% weight, in particular not more than 5% by weight and specifically not more than 2% by weight relative to the total weight of the reaction mixture.

The reactants can in principle be contacted with one another in any desired sequence. For example, the reagent III and the compound II, preferably in neat form or, alternatively, in dissolved or dispersed form, can initially be charged and mixed with each other. The obtained mixture can then be admixed with the catalyst, which is preferably added in neat form or, alternatively, dissolved or dispersed in a suitable solvent, such as a portion of the total amount of the reagent III. Conversely, the catalyst, in neat form or, preferably, dissolved or dispersed in a suitable solvent, such as in particular the total amount of the reagent III or a portion thereof, can be initially charged and admixed with a mixture of the compounds II and III, with the compound II or with a mixture of the compound II and the remaining portion of the reagent III. Alternatively, all reactants can also be added simultaneously to the reaction vessel.

It has been found to be beneficial to initially charge the reaction vessel with a mixture of the reagent III and the compound II preferably in neat form, and then to add the catalyst either in neat form or dissolved in a small portion taken from the total amount of the reagent III.

In general, the reaction of the process according to the invention is performed under temperature control. The reaction is effected in a closed or, preferably, in an open reaction vessel with stirring apparatus or an alternative agitation device. The vessel is preferably also equipped with a condenser allowing the removal of components from the reaction mixture by distillation, such as a Liebig condenser. The reaction temperature of the reaction depends on different factors, in particular on the reactivity of the compound II used and the boiling point of the reagent III, and can be determined by the person skilled in the art in the individual case, for example by simple preliminary tests. In general, the reaction is carried out at a temperature in the range from 20 to 180°C, preferably in the range from 50 to 150°C, more preferably in the range from 70 to 140°C and specifically in the range from 80 to 120°C.

Preferably the reaction of the process according to the invention is carried out at reduced pressure that is typically in the range of 50 to 900 mbar, preferably in the range of 150 to 600 mbar, in particular in the range of 200 to 500 mbar and specifically in the range of 250 to 450 mbar.

In a preferred embodiment of the invention the alcohol R⁵OH that is generated as a byproduct in the reaction between compounds II and III is removed from the reaction mixture during the reaction, in order to push the reaction equilibrium towards the desired product of formula I. The removal of the alcohol R⁵OH is typically accomplished by in-situ distillation which can be readily conducted at reaction temperatures and reaction pressures within the aforementioned preferred ranges. The recovered alcohol R⁵OH, such as ethanol, usually is of good quality and can be recycled.

The work-up of the reaction mixtures obtained in the reaction of the process according to the invention and the isolation of the product of the formula I are effected in a customary manner, for example by an aqueous extractive work-up or by removing not only possibly remaining amounts of volatile byproducts, such as the alcohol R⁵OH, but also the reagent III and possible additional solvents, for example under reduced pressure. The product of formula I may be obtained in sufficient purity by applying such measures or a combination thereof, obviating additional purification steps. Alternatively, further purification can be accomplished by methods commonly used in the art, such as chromatography or distillation.

Preferably the work-up and the isolation of the product of the formula I are effected by removing by evaporation volatile components, such as alcohol R⁵OH that may still be present and possibly an additional solvent, distilling off remaining amounts of the reagent III and finally obtaining the product I in high purity by vacuum distillation. In addition, the reagent of the formula III recovered this way typically is of good quality and, thus, can be recycled.

The compounds of the formula II used in the process according to the invention are well-known in the art and are either commercially available or can readily be obtained by well-established processes, such as the Claisen condensation. For example, ethyl 4,4-difluoro-3-oxobutanoate can be prepared by reacting ethyl 2,2-difluoroacetate with ethyl acetate under basic conditions.

The reagent of the formula III with X being -OR⁵ that is used in the process according to embodiment A, such as trimethyl orthoformate or triethylorthoformate, are well-known in the art and are commercially available. They can be prepared for example by reacting bromoform or chloroform with an alkoxide.

The reagent of the formula III with X being -NR⁶R⁷ that is used in the process according to embodiment B of the invention are known in the art and are either commercially available or can be prepared e.g. by treating the corresponding N,N-dialkylformamide successively with dialkyl sulfate and the corresponding alkoxide, as described for instance by H. Bredereck et al., Angew. Chem. 1961, 73, 493. 1,1-Dimethoxy-N,N-dimethylmethanamine, for example, is accessible by reacting N,N-dimethylformamide initially with dimethyl sulfate and afterwards with sodium methoxide.

The present invention also relates to uses of the halogenated 3-oxocarboxylates carrying a 2-alkoxymethylidene or a 2-dialkylaminomethylidene group of the formula I for preparing 1,3,4-substituted pyrazole compounds, in particular those of formulae (IIIa), (IIIb) or (IIIc): 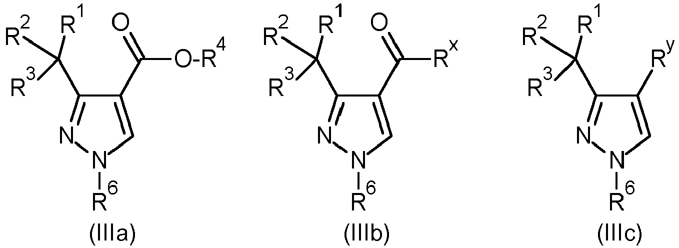 where
- R¹, R², R³ and R⁴: are as defined in any one of claims 1 or 2,
- R⁵: is hydrogen or C₁-C₄-alkyl
- R^{x}: is OH or halogen, and
- R^{y}: is hydrogen or halogen.

These 1,3,4-substituted pyrazole compounds, in particular those of formulae (IIIa), (IIIb) or (IIIc), are key intermediates for the synthesis of a number of valuable pyrazole fungicides mentioned herein at the outset.

The 1,3,4-substituted pyrazole compounds of formula (IIIa) are accessible via cyclisation reaction of a compound of the formula I as the 1,3-difunctional component with a, hydrazine, a C₁-C₄-alkyl hydrazine or C₁-C₄-alkyl hydrazine, such as N-C₁-C₄-alkyl-hydrazone of benzaldehyde. Examples of such reactions are given in US 5,498,624, WO 92/12970, WO 2003/051820, WO 2005/042468, WO 2008/022777 and WO 2009/135808.

Compounds of formula (IIIb) can be obtained by saponification of the compound of formula (IIIa) to yield a compound of formula (IIIb), wherein R^{x} is OH, optionally followed by halogenation, in particular chlorination, to yield a compound of formula (IIIb), wherein R^{x} is halogen, in particular chlorine. Examples of such reactions are given in US 5,498,624, WO 92/12970, WO 2003/051820, WO 2005/042468, WO 2008/022777 and WO 2009/135808.

Compounds of formula (IIIc) can be prepared from compounds of the formula (IIIb), wherein R^{x} is OH, by decarboxylation, e.g. by analogy to the method described in WO 2014/033164, optionally followed by halogenation as described e.g. in WO 2008/145740.

The following examples are intended to serve as further illustration of the invention.

### EXAMPLES

Hereinafter the following abbreviations are used:
TEOF = triethyl orthoformate,
EDFEMAA = ethyl 4,4-difluoro-2-ethoxymethylidene-3-oxobutanoate,
EDFAA = ethyl 4,4-difluoro-3-oxobutanoate,
mol. eq. = molar equivalents,
pTSA = 4-toluenesulfonic acid,
Pyr*pTSA = pyridinium 4-toluenesulfonate,
TEA*pTSA = triethylammonium 4-toluenesulfonate,
MSA = methanesulfonic acid,
TEA*MSA = triethylammonium methanesufonate,
GC = gas chromatography,
area% = proportion of the area of a specific GC peak to the total area of all peaks in percent.

Analytical gas chromatography was carried out under the following conditions: column: Agilent DB-5, 30 m x 0.32 mm, film thickness: 0.25 µm, temperature program: 50°C to 250°C over 10 minutes then 9 minute hold, injection temperature: 50°C, carrier gas: hydrogen, flow: 7.1 psi, detector: flame ionisation detector.

### General procedure for the preparation of ethyl 4,4-difluoro-2-ethoxymethylidene-3-oxobutanoate (EDFEMAA):

To 5.0 g (0.03 mol) of ethyl 4,4-difluoro-3-oxobutanoate (EDFAA) was added triethyl orthoformate (TEOF) and a catalyst (3 mol-%, 0.001 mol). Details on the specific catalyst employed, the amount of the catalyst and the molar equivalents of triethyl orthoformate relative to ethyl 4,4-difluoro-3-oxobutanoate are summarized in Table 1 below for each particular reaction conducted). The mixture was heated to 110°C in an oil bath and the pressure was decreased to 300 mbar, which resulted in a continuous removal of low boilers (mainly ethanol) by distillation. The reaction was continued for 3 hours and afterwards the temperature of the oil bath was reduced to 100°C and the pressure was slowly reduced to 10 mbar to remove remaining TEOF. The reaction mixture is then cooled to ambient temperature and analyzed by gas chromatography. The obtained data on the conversion rate, the amount of the respective acetal intermediate of the formula IV and the purity of the desired product are summarized in Table 1. These data are given in area% of the respective GC peaks unless indicated otherwise. The conversion rate in % is calculated as the percentage share of the peak area of EDFEMAA to the sum of the peak areas of EDFEMAA, EDFAA and the acetal intermediate IV.

GC retention times: EDFAA: 4.97 min, acetal IV: 10,76 min, EDFEMAA (Z- and E-isomers): 11.72 and 11.81 min.

¹H-NMR analysis: The quantitative relationship between the acetal IV and the final product EDFEMAA (Z- and E-isomers combined) can also be determined by comparing the signal integral of the hydrogen at the acetal carbon atom with the added signal integrals of the hydrogens at the methylidene carbon atoms of the Z- and E-isomers.

EDFAA is a compound of the formula II and EDFEMAA is a compound of the formula IA, wherein in each case R¹ is hydrogen, R² and R³ are both fluorine, R⁴ is ethyl and R⁵, if applicable, is ethyl.

**Table 1: Summary of the examples 1 to 13 that are in accordance to the process of the invention and of the comparative examples C1 and C2**

| No. | Catalyst | catalyst [mg] | TEOF [mol. eq.] | Conversion rate¹⁾ [%] | acetal IV²⁾ [area%] | EDFE MAA³⁾ [area %] |
|---|---|---|---|---|---|---|
| 1 | pTSA*H₂O | 170 | 5 | >90 | <1 | >95 |
| 2 | Pyr*pTSA | 227 | 5 | >90 | <1 | >95 |
| 3 | TEA*pTSA | 241 | 5 | >90 | <1 | >95 |
| 4 | MSA | 76 | 5 | >90 | <1 | >95 |
| 5 | TEA*MSA | 178 | 5 | >90 | <1 | >95 |
| 6 | H₃PO₄ (85%) | 88 | 5 | >90 | <1 | >95 |
| 7 | H₃PO₄ (85%) | 88 | 1.5 | >90 | <1 | >90 |
| 8 | H₃PO₄ (85%) | 88 | 1.3 | >90 | <1 | 83⁶⁾ |
| 9 | H₃PO₄ (85%) | 88 | 1.1 | >90 | <1 | 72⁶⁾ |
| 10 | ZnCl₂ | 118 | 5 | >90 | <1 | >95 |
| 11 | AlCl₃ | 120 | 5 | >90 | 55⁵⁾ | 45 |
| 12 | TiCl₄ | 313 | 5 | >90 | 25⁵⁾ | 75 |
| 13 | water⁴⁾ | 16 | 5 | >90 | 42⁵⁾ | 44 |
| C1 | triethylamine | 91 | 5 | >90 | 3 | 19 |
| C2 | none | - | 5 | 90 | 15 | 65 |

| | | | | | | |
|---|---|---|---|---|---|---|
| ¹⁾ calculated from the peak areas as follows: [area EDFEMAA / (area EDFEMAA + area EDFAA + area acetal IV)] x 100, ²⁾ intermediate of the formula IV, ³⁾ Z- and E-isomers combined, ⁴⁾ water was added for the in-situ generation of formic acid. ⁵⁾ it is assumed that these remaining amounts of the acetal IV can essentially completely be converted to EDFEMAA simply by extending the reaction time or by using a higher amount of the catalyst or of water, respectively, ⁶⁾ calculated from ¹H-NMR data. | | | | | | |

As can be seen from Table 1, the process of the invention with the use of a Brønsted acid, an ammonium salt of a Brønsted acid or a Lewis acid in all cases not only give rise to high conversation rates but also to excellent yields of the desired halogenated 2-alkoxymethylidene-3-oxocarboxylates of the formula IA. In contrast, the comparative examples C1 and C2 carried out without adding a catalyst or with a base as catalyst resulted in substantially lower yields and in the latter case also in an insufficient conversation rate.

## Claims

1. A process for the preparation of a compound of the formula I, 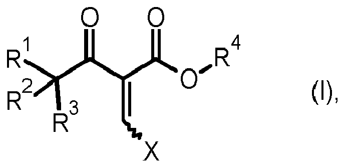 wherein
R¹ and R² are each independently from one another selected from hydrogen, fluorine, bromine, chlorine and trifluoromethyl,
R³ is selected from fluorine chlorine and trifluoromethyl,
R⁴ is selected from C₁-C₄-alkyl, and
X is either -OR⁵ or -NR⁶R⁷, wherein R⁵ is selected from C₁-C₄-alkyl, and R⁶ and R⁷ are each independently from one another selected from C₁-C₄-alkyl,
which comprises the reaction of a compound of the formula II 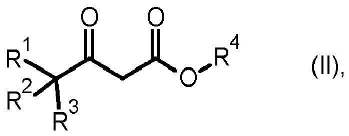 with a reagent of the formula III, 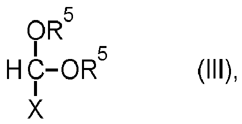 wherein the variables R¹, R², R³, R⁴, R⁵ and X given in formulae II and III have the above defined meanings,
in the presence of a catalyst selected from Brønsted acids, ammonium salts of Brønsted acids and Lewis acids;
wherein the reaction is performed essentially in the absence of a carboxylic acid anhydride.

2. The process according to claim 1, wherein
R¹ is selected from hydrogen, fluorine and chlorine,
R² is selected from hydrogen, chlorine and trifluoromethyl,
R³ is fluorine, and
R⁴ is methyl or ethyl.

3. The process according to claim 2, wherein
R¹ is fluorine and R² is hydrogen; or
R¹ is fluorine and R² is trifluoromethyl.

4. The process according to any one of the preceding claims, wherein X is -OR⁵ and R⁵ is identical with R⁴.

5. The process according to any one of claims 1 to 3, wherein X is -NR⁶R⁷ with R⁶ and R⁷ being each independently from one another selected from methyl and ethyl.

6. The process according to any one of the preceding claims, wherein the combined quantities of the compounds of formulae I, II and III, including ketals, semiketals and hydrate of compound of formula II, amount for at least 75% by weight, preferably at least 80% by weight, of the reaction mixture at the beginning of the reaction.

7. The process according to any one of the preceding claims, wherein the catalyst is a Brønsted acid or an ammonium salt of a Brønsted acid.

8. The process according to claim 7, wherein the catalyst is selected from the group consisting of C₁-C₄-alkanoic acids, such as acetic acid and aryl carboxylic acids, such as benzoic acid.

9. The process according to claim 7, wherein the catalyst is selected from the group consisting of C₁-C₄-alkyl sulfonic acids, aryl sulfonic acids, cycloaliphatic sulfonic acids, oxoacids of phosphor and ammonium salts thereof.

10. The process according to claim 9, wherein the catalyst is selected from the group consisting of methansulfonic acid, benzenesulfonic acid, 4-toluenesulfonic acid, camphorsulfonic acid, pyridinium 4-toluenesulfonate, trimethylammonium 4-toluenesulfonate and trimethylammonium methansulfonate.

11. The process according to claim 9, wherein the catalyst is H₃PO₄.

12. The process according to any one of claims 1 to 6, wherein the catalyst is a Brønsted acid that is generated in situ by adding water to the reaction mixture.

13. The process according to any one of claims 1 to 6, wherein the catalyst is a Lewis acid selected from ZnCl₂, FeCl₃, TiCl₄ and AlCl₃.

14. The process according to any one of the preceding claims, wherein the molar ratio of the orthoformate of the formula III to the compound of formula II is from 1:1 to 10:1.

15. The process according to any one of the preceding claims, which has one, two, three or four of the following features i) to iv):
i) the molar ratio of the catalyst to the compound of formula II is from 0.0001:1 to 0.1:1,
ii) the reaction is carried out at a temperature in the range of 20 to 180°C,
iii) the reaction is carried out at reduced pressure that is preferably in the range of 50 to 900 mbar
iv) the alcohol of the formula R⁵OH which is formed during the reaction is removed, at least partially, from the reaction mixture in the course of the reaction, preferably by distillation.

16. The use of the process of any of the preceding claims for preparing a pyrazole compound of the formulae (IIIa), (IIIb) or (IIIc): 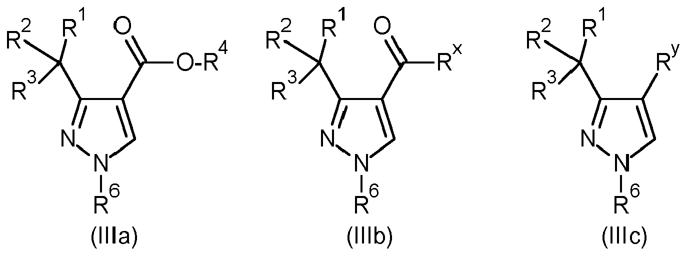 where
R¹, R², R³ and R⁴ are as defined in any one of claims 1 or 2,
R⁵ is hydrogen or C₁-C₄-alkyl,
R^{x} is OH or halogen, and
R^{y} is hydrogen or halogen.
